Europäisches Patentamt

European Patent Office

Office européen des brevets

(19)

(11) Veröffentlichungsnummer : **0 027 172**
**B1**

(12) # EUROPÄISCHE PATENTSCHRIFT

(45) Veröffentlichungstag der Patentschrift :
22.06.83

(51) Int. Cl.³ : **A 61 F 13/00**, A 61 F 13/06,
A 61 F 13/10

(21) Anmeldenummer : **80105178.0**

(22) Anmeldetag : **30.08.80**

(54) **Bandage.**

(30) Priorität : 07.09.79 DE 2936174

(43) Veröffentlichungstag der Anmeldung :
22.04.81 Patentblatt 81/16

(45) Bekanntmachung des Hinweises auf die Patenterteilung : 22.06.83 Patentblatt 83/25

(84) Benannte Vertragsstaaten :
AT BE CH DE FR GB IT LI NL SE

(56) Entgegenhaltungen :
CH A 152 404
DE A 2 551 847
DE U 1 929 624
US A 2 524 326
US A 3 194 233
US A 3 318 305
US A 3 458 867
US A 3 831 467
US A 3 934 583

(73) Patentinhaber : **Bauerfeind Gesellschaft mit beschränkter Haftung**
**Wiesenstrasse 4-16**
**D-4152 Kempen 1 (DE)**

(72) Erfinder : **Hess, Heinrich, Prof. Dr.**
**Kapuzinerstrasse 4**
**D-6630 Saarlouis (DE)**
Erfinder : **Bauerfeind, Hans B.**
**Wiesenstrasse 18**
**D-4152 Kempen - 1 (DE)**
Erfinder : **Kleylein, Horst**
**Sudetenstrasse 13**
**D-8502 Zirndorf/Nürnberg (DE)**

(74) Vertreter : **Stark, Walter, Dr.-Ing.**
**Moerser Strasse 140**
**D-4150 Krefeld (DE)**

EP 0 027 172 B1

Jouve, 18, rue St-Denis, 75001 Paris, France

Bandage

Die Erfindung betrifft eine Bandage aus elastischem Bandagenstoff, insbesondere in Schlauchform für die Abstützung bzw. Kompression von Knie-, Sprung-, Ellenbogen- und/oder Handgelenken, mit wenigstens einer im angelegten Zustand die Knochenvorsprünge des Gelenkes umgebenden, die benachbarten Gelenkweichteile beaufschlagenden Kompressionseinlage. Derartige Bandagen werden bei Gelenkverletzungen, wie sie insbesondere beim Sport vorkommen, aber auch bei degenerativen Gelenkveränderungen verwendet.

Die bekannten Bandagen (US-A-3 458 867) sind meist schlauch- oder strumpfförmig aus elastischen Gummi- oder Kunststofffäden mit einer Kompressionseinlage aus Schaumkunststoff hergestellt und werden bei einer Gelenkverletzung über das betreffende Gelenk gezogen. Aufgrund ihrer Elastizität üben sie eine stützende und gleichzeitig massierende Wirkung aus, die dem Abbau (Resorption) des sich bei derartigen Verletzungen fast immer bildenden entzündlichen Ergusses dienen soll.

Die Kompressionseinlage aus Schaumkunststoff verhält sich wie eine Anordnung von auf einer Fläche verteilten einzelnen Federn. Diejenigen Bereiche des Schaumkunststoffes, die nicht belastet werden, erfahren auch keine Kompression, sie bleiben von den Druckwirkungen der anderen Bereiche unberührt und ändern auch ihre räumliche Lage nicht. Deswegen wird bei den bekannten Bandagen zwar auf die unmittelbaren Druckflächen eine Belastung ausgeübt, die bei Belastungen auch eine Wechselbelastung sein kann, benachbarte Bereiche bleiben aber unberührt, so daß deren Durchblutung nach wie vor unbefriedigend bleibt.

Der Erfindung liegt die Aufgabe zugrunde, eine Bandage so zu gestalten, daß bei Gelenkverletzungen ein schnelleres Abschwellen und eine bessere Resorption des damit verbundenen Ergusses und zudem ein angenehmeres Tragen bewirkt wird.

Diese Aufgabe wird erfindungsgemäß dadurch gelöst, daß die Kompressionseinlage aus elastischem, jedoch inkompressiblem Silikonkautschuk oder einem Material mit gleichen Elastizitäts- und Kompressionseigenschaften besteht.

In Ausbildung der Erfindung ist vorgesehen, daß die Kompressionseinlage ringförmig ausgebildet ist, so daß die gesamten Weichteile des jeweiligen Gelenkes von der Kompressionseinlage beaufschlagt werden. Dabei ist es für gewisse Anwendungszwecke von Vorteil, wenn an der der Kompressionseinlage gegenüberliegenden Seite eine weitere Kompressionseinlage angeordnet wird.

Als ein günstiges Maß für die Kompressionseinlage hat sich eine Dicke von etwa 3 bis 4 mm erwiesen.

Da die Kompressionseinlage aus elastischem, jedoch inkompressiblem Silikonkautschuk besteht, entsteht ein Rolleffekt, der eine intensive Massage der Gelenkweichteile bewirkt.

In der Zeichnung ist die Erfindung anhand eines Ausführungsbeispieles näher veranschaulicht. Es zeigen :

Figur 1 eine Bandage mit Kompressionseinlage und

Figur 2 die Bandage gemäß Fig. 1 in angelegtem Zustand.

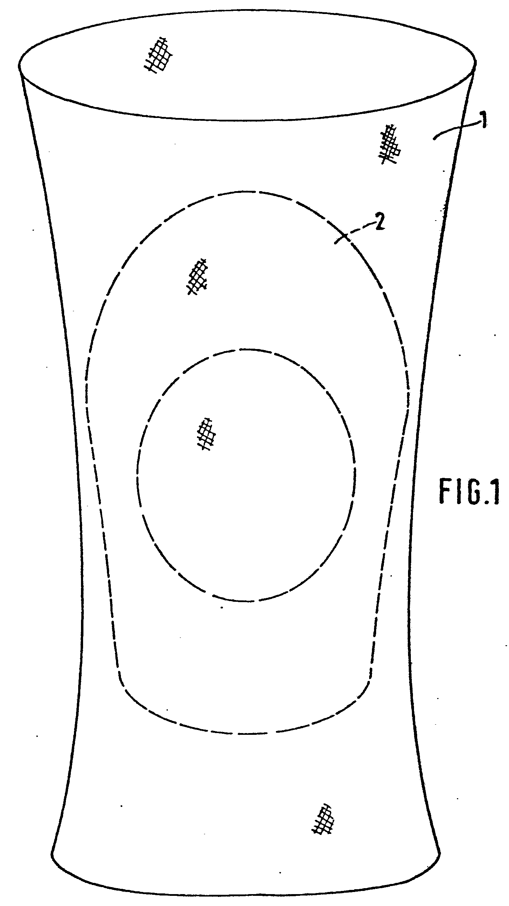

In Figur 1 ist eine schlauchförmige Bandage 1 aus Bandagengewebe für ein Kniegelenk dargestellt. Sie weist eine ringförmige Kompressionseinlage 2 von etwa 3 bis 4 mm Dicke auf, die so geformt ist, daß sie im angelegten Zustand nicht die Kniescheibe selbst beaufschlagt, sondern ausschließlich die dieser benachbarten Gelenkweichteile.

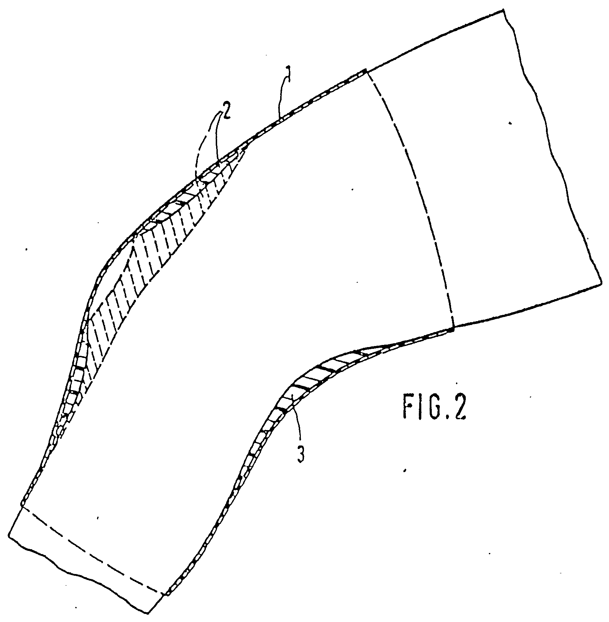

Dies geht insbesondere aus Figur 2 hervor, die die Bandage 1 im angelegten Zustand zeigt. Die ringförmige Kompressionseinlage 2 umgibt die nur durch das Bandagengewebe abgedeckte Kniescheibe, so daß die Gelenkweichteile kompremiert und damit massiert werden. Auf die Kniescheibe selbst wird praktisch kein Druck mehr ausgeübt, so daß die Gelenkfunktion voll erhalten bleibt. Zur Unterstützung ist an der Hinterseite der Bandage 1 noch ein Gelenkpolster 3 eingesetzt.

Diese Bandage 1 bewirkt ein wesentlich schnelleres Abschwellen und eine verbesserte Resorption der Ergüsse und zeichnet sich darüber hinaus durch angenehmeres Tragen aus, da sie praktisch nicht mehr einschnürt.

## Ansprüche

1. Bandage aus elastischem Bandagenstoff, insbesondere in Schlauchform für die Abstützung bzw. Kompression von Knie-, Sprung-, Ellenbogen- und/oder Handgelenken, mit wenigstens einer im angelegten Zustand die Knochenvorsprünge des Gelenks umgebenden, die benachbarten Gelenkweichteile beaufschlagenden Kompressionseinlage, dadurch gekennzeichnet, daß die Kompressionseinlage (2) aus elastischem, jedoch inkompressiblem Silikonkautschuk oder einem Material mit gleichen Elastizitäts- und Kompressionseigenschaften besteht.

2. Bandage nach Anspruch 1, dadurch gekennzeichnet, daß die Kompressionseinlage (2) ringförmig ausgebildet ist.

3. Bandage nach einem der Ansprüche 1 oder 2, dadurch gekennzeichnet, daß an der der Kompressionseinlage (2) gegenüberliegenden Seite eine weitere Kompressionseinlage oder ein Gelenkpolster (3) angeordnet ist.

4. Bandage nach einem der Ansprüche 1 bis 3, dadurch gekennzeichnet, daß die Kompressionseinlage (2) 3 bis 4 mm dick ist.

## Claims

1. A bandage formed from elastic material, particularly in the form of a tube for supporting or compressing knee-, ankle-, elbow- and/or wrist-joints, and having at least one compression insert which, in use, surrounds the bone projections of the joint and acts upon the adjacent soft parts of the joint, characterised in that the compression insert (2) comprises an elastic, but incompressible silicon caoutchouc or a material having the same elasticity and compression properties.

2. A bandage according to claim 1, characterised in that the compression insert (2) has an annular shape.

3. A bandage according to claim 1 or 2, characterised in that a further compression insert or a joint cushion (3) is arranged on the side opposite the compression insert (2).

4. A bandage according to any one of claims 1 to 3, characterised in that the compression insert (2) is 3 to 4 mm thick.

**Revendications**

1. Bandage en matière élastique de bandage, en particulier sous forme tubulaire, pour le soutien ou la compression des articulations du genou, du cou-de-pied, du coude et/ou de la main, comportant au moins un doublage de compression qui, dans l'état appliqué, entoure les saillies osseuses de l'articulation et agit sur les parties molles adjacentes des tissus, caractérisé en ce que le doublage de compression (2) est formé de caoutchouc de silicone élastique mais incompressible ou d'une matière ayant des propriétés d'élasticité et de compression semblables.

2. Bandage selon la revendication 1, caractérisé en ce que le doublage de compression (2) est de forme annulaire.

3. Bandage selon l'une des revendications 1 et 2, caractérisé en ce que du côté opposé au doublage de compression (2) est disposé un autre doublage de compression ou un coussin d'articulation (3).

4. Bandage selon l'une des revendications 1 à 3, caractérisé en ce que le doublage de compression (2) a une épaisseur de 3 à 4 mm.

FIG.1

FIG.2